# EUROPEAN PATENT SPECIFICATION

(11) **EP 0 892 646 B1**
(45) Date of publication and mention of the grant of the patent: **18.12.2002**
(21) Application number: 97915210.5
(22) Date of filing: 09.04.1997
(51) Int. Cl.: A61L 9/12

(54) **A METHOD AND APPARATUS FOR SCENTING ROOMS IN A PROGRAMMED MANNER**
VERFAHREN UND VORRICHTUNG ZUR RAUMBEDUFTUNG IN EINER PROGRAMMIERTEN EINRICHTUNG
PROCEDE ET APPAREIL PERMETTANT DE PARFUMER DES PIECES DE MANIERE PROGRAMMEE

(30) Priority: 09.04.1996 BR 9601523
(43) Date of publication of application: 27.01.1999
(73) Proprietor: Araujo De Sousa, Mauricio, 05065-001 Sao Paulo, SP (BR)
(72) Inventor: Araujo De Sousa, Mauricio, 05065-001 Sao Paulo, SP (BR)
(74) Representative: Nettleton, John Victor
(86) International application number: PCT/BR97/00012
(87) International publication number: WO 97/037693

(56) References cited:
- EP-A- 0 508 939
- WO-A-94/09493
- DE-A- 4 033 076
- DE-A- 19 513 293
- FR-A- 2 553 666
- US-A- 4 603 030
- US-A- 5 069 876
- DATABASE WPI Section PQ, Week 8429 Derwent Publications Ltd., London, GB; Class P34, AN 84-177169 XP002037302 & AU 21442 83 A (WALLACE R W) , 31 May 1984
- PATENT ABSTRACTS OF JAPAN vol. 017, no. 471 (P-1601), 26 August 1993 & JP 05 109253 A (TOSHIBA CORP), 30 April 1993,
- PATENT ABSTRACTS OF JAPAN vol. 010, no. 197 (E-418), 10 July 1986 & JP 61 041229 A (TAMAPATSUKU KK), 27 February 1986,
- PATENT ABSTRACTS OF JAPAN vol. 016, no. 257 (C-0949), 11 June 1992 & JP 04 058956 A (MATSUSHITA ELECTRIC IND CO LTD), 25 February 1992,

## Description

### FIELD OF THE INVENTION

The present invention refers to a method and apparatus for scenting rooms in a programmed manner.

More specifically, it relates to a method and apparatus for scenting, in a programmed way closed spaces or environments, such as TV rooms, video rooms, CD-ROM rooms, movie theaters, playhouse auditoria and the like.

Even more specifically, the present invention refers to a method and apparatus designed for scenting rooms, associated and synchronized with a video tape, a motion picture, a CD-ROM disk, a theatrical play, or even with the plot of a musical.

### BACKGROUND OF THE INVENTION

Methods, apparatus and devices for scenting rooms are much diversified and widely commercialized at present. Thus, for instance, there are aerosol containers for scenting environments, devices for holding sticks or tablets intended to scent bathrooms, apparatus designed to be connected to water pipes for flushing toilets and urinals. All these devices for dispensing scents into the room provide a wide range of fragrances such as lavender, wild flowers, tutti-frutti, green apple and others.

In addition to these state of the art elements, some patent documents disclose methods and dispensing devices for scenting rooms, including the release of different fragrances in a pre-determined sequence. An example of such documents is US Patent 5,069,876, published on December 3, 1991, which discloses a display unit for selling products which basically includes an audio unit associated to a scent-dispensing device. Such a unit can also be associated to a photograph display unit. The functioning of such units can be associated in a pre-selected manner. Patent application FR 2,553,666, published on April 26, 1985, teaches an individual, portable device for dispensing perfume fragrances in connection with the presentation of pictures and sound. US Patent 4,603,030, published on July 29, 1986, relates to a system for dispensing a variety of different fragrances in response to a predetermined and programmed sequence of fragrances, with a predetermined duration. Patent application EP 0 295 129, published on December 14, 1988, discloses a method and apparatus for dispensing different fragrances into an environment from different storage containers, in accordance with a predetermined timing controlled by a timer. US Patent 5,342,584, published on August 30, 1994, disclosed a battery-driven device provided with a cartridge impregnated with a fragrance for dispensing at least two different scents. US Patent 5,273,690, published on December 28, 1993, discloses a device employing compressed air together with a carrier charged with a variety of fragrances placed in individual cells. These cells have breakable walls for releasing the scent into the current of compressed air. US Patent 5,178,327, published on January 12, 1993, discloses an apparatus for scenting rooms including a range of different aromas that are selected by means of a rotating cylinder or disk. UK Patent application 2 256 589, published on December 16, 1992, discloses a device for scenting rooms having multiple compartments for different aromas. Patent application UK 2 249 958, published on May 27, 1992, discloses a device for generating aromas, which are selected by a user from a distance.

However, it has not yet been proposed an effective method for scenting rooms that works timed and tuned to the event during its performance. The event in question is, for instance, the presentation of a movie on television, a video cassette tape, a CD-ROM disk, a motion picture in a movie theater, a stage play or an artistic show and/or a musical.

### OBJECTIVES OF THE INVENTION

Thus, a first objective of the invention is to provide a method for scenting rooms that is tuned to and synchronized with an event at the time of its performance.

Another object of the present invention is to provide a method for scenting rooms or environments, preferably closed spaces such as rooms for television, video and CD-ROM, movie houses, theaters and playhouse auditoria, the scenting being carried out in tune and synchronization with the motion picture or event while the latter is being presented.

A further objective of the present invention is to provide an apparatus for carrying out the method of the invention.

### SUMMARIZED DESCRIPTION OF THE INVENTION

According to one aspect of the present invention, a method is provided by which a room is scented with several different fragrances, in a sequence following specifically the plot of an event, such as a motion picture, a play or a show, the fragrance spreading throughout the room where the event is taking place, so as to provide the audience with the sensation of experiencing that moment or fact together with the protagonist(s) of the event.

According to a second aspect of the present invention, an apparatus is provided for carrying out the aforementioned method, said apparatus including a carrier having several individual compartments containing various fragrances to be dispensed into the room in which the event takes place.

### BRIEF DESCRIPTION OF THE DRAWINGS

The present invention will be better understood with reference to the accompanying drawings, which do not limit the scope of the invention described here and are to be seen only as an illustration of an embodiment of the invention.
- Figure 1 is a perspective view of the apparatus of the invention;
- Figure 2 is a cross-section view of the apparatus of the invention;
- Figure 3 is a top view of the carrier element for the scenting compartments; and
- Figure 4 is a top view of the movie theater equiped with apparatus in accordance with the present invention.

### DETAILED DESCRIPTION OF THE INVENTION

According to the present invention, a method is provided for scenting rooms or environments, preferably closed spaces, by which a sequence of fragrances are dispersed into said environment, tuned to and synchronized with an event that is being presented, said event being a motion picture, a play or an artistic show and/or a musical. The sequence of fragrances dispersed into the room will be effected by means of a carrier of scenting substances or compositions, which will be operated tuned to the beginning of the event and in synchronization with the actions, places and movements performed during the event. This feature will provide the audience with a feeling of greater reality during the various scenes viewed, since the spectators will be able to smell every scent characteristic of the location where the scene takes place.

Scenting substances and compositions to be used in the method of the present invention should be either produced or formulated in accordance with the standards established for this type of product which will be inhaled by people. Thus, they must be non-toxic, anti-allergic, non-irritant and as inert as possible with respect to the environment. The possible number of formulations to be used in the method of the present invention can be unlimited, however, it will be appreciated that such formulations should include at least those which imitate all the most frequent odours perceived daily in our environment, such as the smell of rain, wet soil, garden flowers, dust, forest, etc. However, fetid odours, if used, should be avoided or at least attenuated. Such odoriferous substances or compositions may be powdered formulations, granules or liquids, and so they must be adequately packed in accordance with their nature. The packages should be individual and should contain defined amounts of scenting substances or compositions, so that they can be easily broken open in the established sequence to supply the scent during a pre-established period of time.

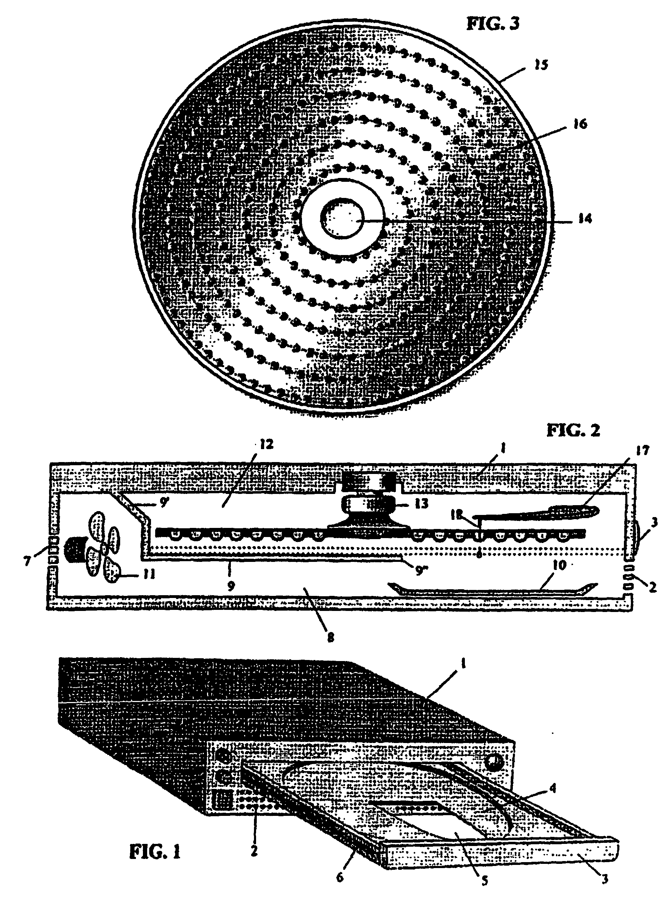

Figures 1, 2 and 3 show an example of an apparatus for carrying out the method of the present invention, which comprises a generally rectangular housing (1), which has several small bores (2) at its front portion, longitudinally disposed along its length and several small bores (7) at its back portion. Above said bores (2) there is a movable receptable (3), provided with a circular recess (4), which has a hollowed-out portion (5). Said movable receptable (3) is provided with side guides (6), so that it can slide upon being opened and closed.

The housing (1) encloses lower (8) and upper (12) compartments, divided by an inclined plate (9) which extends from the back portion (9') of said housing (1) to an intermediate portion (9"), forming a channel in said compartment (8) and a housing for a mechanism (13). This compartment (8) has in its back portion a space where a small fan (11) is installed facing a receptable (10) located at the front portion of said compartment (8) of the housing (1).

Said mechanism (13) is of the type which fits into the central bore (14) of a disk (15) carrying small capsules of fragrances (16) and is complemented by a a device that moves in the vertical direction (17), provided with a pointed element (18) for perforating said capsules (16) containing fragrances. For this purpose, said capsules (16) have to be made from a material suitable to be punctured by said pointed element (18).

The above-described apparatus is electronic and works in response to signals received from the video tape, CD-ROM disk or a pre-established software, as for instance, from a microcomputer. Thus, the disk (15) containing a given sequence of capsules (16) of fragrances, pre-established in tune with the event, movie or show, is adequately inserted into the receptable (3), which is then closed. At the beginning of the event the apparatus is turned on and then, in synchronism with the sequence of scenes presented and when a determined fragrance is required, the mechanism (13) turns the disk (15), positioning the respective capsule (16) of the required fragrance under the device (17), which, with a vertical movement downwards and upwards, in this order, perforates said capsule (16), thus tearing it open with the pointed element (18). At this moment the scenting substance or composition contained in the capsule (16) falls onto the receptacle (10) and the small fan (11) is immediately driven and sucks air through the small back bores (7), thus creating a current of air in the compartment (8) under the plate (9) and exhaling the respective fragrance through the small front bores (2). In this way, the fragrance is transferred to the room where the event is taking place and spreads out therein creating therein an atmosphere of reality for the spectators. This procedure is repeated by the apparatus, which will select the capsule to be opened at the next scene requiring a specific fragrance.

The duration of the fragrance can be controlled, for instance, by the chemical formulation itself, previously produced for the respective event. The duration of the fragrance can also be controlled by means of odor-inhibiting agents, such as chemical inhibitors, which can be foreseen in the set of capsules (16). Thus, each event will have its disk with the corresponding sequence of capsules of fragrance. However, this does not exclude a random supply of fragrances for a given event.

The driving of the apparatus as a whole, that is to say, the mechanism (13), the puncturing device (17) and the fan (11), is effected by electronic signals, inaudible to the spectators. In the same way, the means which actuate these elements are such as not to produce any sounds or noises strange to the ear during the performance of the event.

The method and apparatus of the present invention can be produced for most diverse situations. Thus, for instance, they can be designed for existing movies, or else program them for synchronization with the sequence of the scenes. Old movies can be reedited with signals for synchonism with the inventive apparatus, or else tapes incorporating signals for synchronism with the apparatus can be made.

The disks produced can be either disposable or returnable to be re-edited. This edition is obviously accomplished by refilling them with respective fragrance capsules referring specifically to the corresponding motion picture.

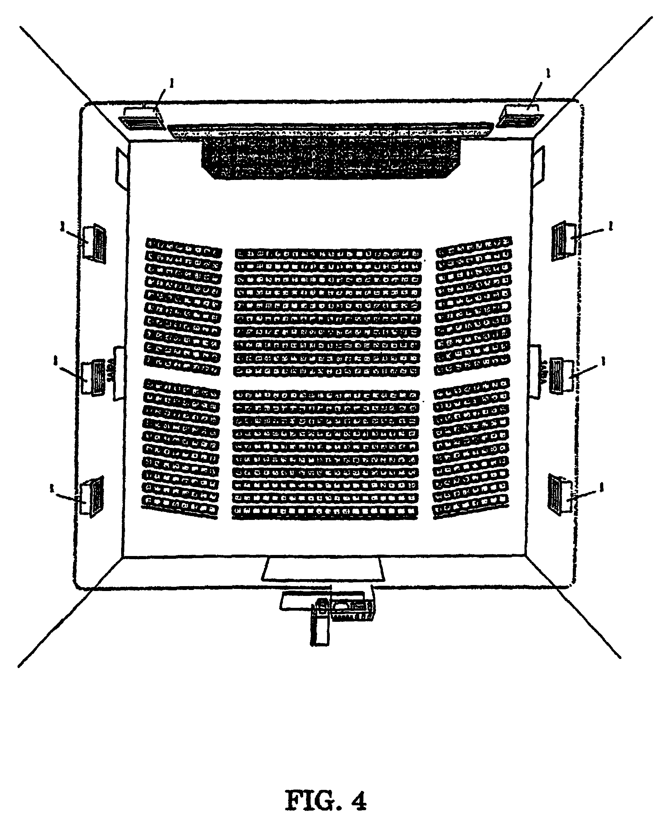

The apparatus of the present invention is usually small and can be installed, for instance, besides a television set, a video cassete recorder or a microcomputer with CD-ROM. As can be seen from figure 4, several units of the apparatus (1) can be conveniently distributed in large rooms such as movie theaters and, in this case, they will be driven at the same time and will have the same disks. The apparatus of the present invention can even be produced incorporated in television sets, as is the case with the current video cassette recorders.

## Claims

1. A method for exhaling a sequence of fragrances into a room tuned to and synchronised with an event, said method being **characterised by** comprising the steps of:
sequentially selecting one capsule of a set of fragrance-filled capsules (16) in response to an electronic signal associated with an action of the event, said capsules being disposed on a turning disk (15) inside a housing (1);
opening the selected capsule and dropping the fragrance of said capsule on a receptacle (10) located below said turning disk; and
creating an air current inside said housing, whereby said fragrance is exhaled outwards of the housing into said room.

2. A method according to claim 1, **characterised in that** said capsules (16) are opened by perforation.

3. A method according to claim 1, **characterised in that** said air current is created by means of a fan (11) located inside said housing.

4. A method according to claim 1, 2 or 3, **characterised in that** said capsules (16) are opened in response to an electronic signal received from a video tape, or from a CD-room disk, or from a software of a microcomputer.

5. An apparatus for carrying out the method defined in anyone of the preceding claims, **characterised by** comprising:
a housing (1) having on a first side and on a second side opposite to said first side a plurality of small bores (2, 7);
a movable receptacle (3) in said housing, said receptacle (3) having a hollowed out portion (5);
a turning disk (15) above said receptacle (3), said disk being capable of carrying a set of fragrance-filled capsules (16):
an opening means (18) for opening a predetermined capsule (16) above said hollowed out portion, said capsule being positioned above said hollowed out portion by means of rotation of said turning disk (15) in response to an electronic signal;
a receptacle (10) below said movable receptacle (3) for receiving the fragrance dropped from said capsule; and
a fan (11) facing said receptacle (10), said fan being activated upon receipt of said electronic signal, thereby exhaling said fragrance through said a plurality of small bores (2) of said first side.

6. An apparatus according to claim 5. **characterised in that** said opening means comprises a pointed element (18), said pointed element being movable in a vertical direction above said disk.

7. An apparatus according to claim 5, **characterised in that** said housing (1) is divided into upper and lower compartments (12,8) by a plate (9) that extends from said second side toward said first side.

8. An apparatus according to claim 7, **characterised in that** said movable receptacle (3) and said turning disk (15) are located in said upper compartment (12), while said receptacle (10) and said fan (11) are located in said lower compartment (8).

9. An apparatus according to claim 5, 6, 7 or 8, **characterised in that** said capsules (16) are opened in response to an electronic signal received from a video tape, or from a CD-room disk, or from a software of a microcomputer.

## Patentansprüche

1. Verfahren zum Ausgeben einer Sequenz von Duftstoffen in einen Raum, in Abstimmung und Synchronisation mit einem Ereignis, wobei dieses Verfahren **gekennzeichnet ist durch** folgende Schritte:
sequenzielles Auswählen einer Kapsel aus einem Satz duftstoffgefüllter Kapseln (16) als Antwort auf ein elektronisches Signal, das mit einer Aktion des Ereignisses verknüpft ist, wobei die Kapseln auf einer Drehscheibe (15) innerhalb eines Gehäuses (1) angeordnet sind;
Öffnen der ausgewählten Kapsel und Abgeben des Duftstoffes der Kapsel auf einen Behälter (10), der unterhalb der Drehscheibe angeordnet ist; und
Erzeugen eines Luftstroms innerhalb des Gehäuses, wodurch der Duftstoff aus dem Gehäuse heraus in den Raum ausgegeben wird.

2. Verfahren nach Anspruch 1, **dadurch gekennzeichnet, dass** die Kapseln (16) durch Perforieren geöffnet werden.

3. Verfahren nach Anspruch 1, **dadurch gekennzeichnet, dass** der Luftstrom mittels eines Ventilators (11) erzeugt wird, der innerhalb des Gehäuses angeordnet ist.

4. Verfahren nach Anspruch 1, 2 oder 3, **dadurch gekennzeichnet, dass** die Kapseln (16) in Antwort auf ein elektronisches Signal geöffnet werden, das von einem Videoband, von einer CD-ROM-Scheibe oder von einer Software eines Mikrocomputers empfangen wird.

5. Vorrichtung zum Durchführen des Verfahrens, wie es in irgendeinem der vorangehenden Ansprüche definiert ist, **gekennzeichnet durch** Folgendes:
ein Gehäuse (1), das auf einer ersten Seite und auf einer zweiten, der ersten Seite gegenüberliegenden Seite eine Mehrzahl kleiner Bohrungen (2, 7) aufweist;
einen beweglichen Behälter (3) in dem Gehäuse, wobei dieser Behälter (3) einen ausgehöhlten Teil (5) aufweist;
eine Drehscheibe (15) oberhalb dieses Behälters (3), wobei diese Drehscheibe imstande ist, einen Satz duftstoffgefüllter Kapseln (16) zu tragen;
ein Öffnungsmittel (18) zum Öffnen einer vorbestimmten Kapsel (16) über dem ausgehöhlten Teil, wobei diese Kapsel über diesen ausgehöhlten Teil mittels Drehung der Drehscheibe (15) in Antwort auf ein elektronisches Signal platziert wird;
einen Behälter (10) unter dem beweglichen Behälter (3) zum Aufnehmen des aus der Kapsel ausgegebenen Duftstoffes; und
einen Ventilator (11), der auf den Behälter (10) ausgerichtet ist, wobei dieser Ventilator bei Empfang des elektronischen Signals aktiviert wird, wodurch der Duftstoff **durch** die Mehrzahl der kleinen Bohrungen (2) an der ersten Seite ausgegeben wird.

6. Vorrichtung nach Anspruch 5, **dadurch gekennzeichnet, dass** das Öffnungsmittel ein spitzgeformtes Element (18) aufweist, und dass das spitzgeformte Element in einer vertikalen Richtung oberhalb der Scheibe bewegbar ist.

7. Vorrichtung nach Anspruch 5, **dadurch gekennzeichnet, dass** das Gehäuse (1) in ein oberes und ein unteres Abteil (12, 8) mittels einer Platte (9), die sich von der zweiten Seite zur ersten Seite hin erstreckt, geteilt ist.

8. Vorrichtung nach Anspruch 7, **dadurch gekennzeichnet, dass** der bewegliche Behälter (3) und die Drehscheibe (15) in dem oberen Abteil (12) angeordnet sind, während der Behälter (10) und der Ventilator (11) in dem unteren Abteil (8) angeordnet sind.

9. Vorrichtung nach Anspruch 5, 6, 7 oder 8, **dadurch gekennzeichnet, dass** die Kapseln (16) in Antwort auf ein elektronisches Signal geöffnet werden, das von einem Videoband, von einer CD-ROM-Scheibe oder von einer Software eines Mikrocomputers empfangen wird.

## Revendications

1. Procédé pour exhaler une séquence de parfums dans un local, accordé à et synchronisé avec un événement, ledit procédé étant **caractérisé par** le fait de comprendre les étapes consistant à :
sélectionner séquentiellement une capsule d'un jeu de capsules remplies de parfum (16) en réponse à un signal électronique associé à une action de l'événement, lesdites capsules étant disposées sur un disque (15) rotatif, à l'intérieur d'un boîtier (1) ;
ouvrir la capsule sélectionnée, et
faire tomber le parfum de ladite capsule sur un réceptacle (10) placé au-dessous dudit disque rotatif ; et
créer un courant d'air à l'intérieur dudit boîtier, de manière que ledit parfum soit émis à l'extérieur du boîtier, dans ledit local.

2. Procédé selon la revendication 1, **caractérisé en ce que** lesdites capsules (16) sont ouvertes par perforation.

3. Procédé selon la revendication 1, **caractérisé en ce que** ledit courant d'air est créé au moyen d'un ventilateur (11) placé à l'intérieur dudit boîtier.

4. Procédé selon la revendication 1, 2 ou 3, **caractérisé en ce que** lesdites capsules (16) sont ouvertes en réponse à un signal électronique reçu d'une bande vidéo, ou bien depuis un disque CD-ROM ou bien depuis un logiciel sur un micro-ordinateur.

5. Dispositif de mise en oeuvre du procédé défini selon l'une quelconque des revendications précédentes, **caractérisé en ce qu'**il comprend :
un boîtier (1) ayant une pluralité de petits trous (2, 7) sur un premier côté et sur un deuxième côté opposé audit premier côté ;
un réceptacle (3) mobile dans ledit boîtier, ledit réceptacle (3) ayant une partie extérieure (5) évidée par creusement ;
un disque rotatif(15) au-dessus dudit réceptacle (3), ledit disque étant capable de porter un jeu de capsules (16) remplies par du parfum ;
des moyens d'ouverture (18) pour ouvrir une capsule (16) prédéterminée située au-dessus de ladite partie creusée, ladite capsule étant positionnée au-dessus de ladite partie creusée par la rotation dudit disque rotatif (15), en réponse à un signal électronique ;
un réceptacle(10) placé au-dessous dudit réceptacle mobile(3), afin de recevoir le parfum tombé depuis ladite capsule ; et
un ventilateur(11) faisant face audit réceptacle (10), ledit ventilateur étant activé lors de la réception dudit signal électronique, de manière à émettre ledit parfum par ladite pluralité de petits trous (2) situés dudit premier côté.

6. Dispositif selon la revendication 5, **caractérisé en ce que** lesdits moyens d'ouverture comprennent un élément pointu (18), ledit élément pointu étant mobile dans une direction verticale au-dessus dudit disque.

7. Dispositif selon la revendication 5, **caractérisé en ce que** ledit boîtier (1) est divisé en compartiments supérieur et inférieur (12, 8), par une plaque (9) qui s'étend depuis ledit deuxième côté vers ledit premier côté.

8. Dispositif selon la revendication 7, **caractérisé en ce que** ledit réceptacle mobile(3) et ledit disque rotatif(15) sont placés dans ledit compartiment supérieur (12), tandis que ledit réceptacle (10) et ledit ventilateur (11) sont placés dans ledit compartiment inférieur (8).

9. Dispositif selon la revendication 5, 6, 7 ou 8, **caractérisé en ce que** lesdites capsules (16) sont ouvertes en réponse à un signal électronique reçu depuis une bande vidéo ou depuis un disque CD-rom, ou depuis un logiciel d'un micro-ordinateur.
